# EUROPEAN PATENT SPECIFICATION

(11) **EP 4 603 067 B1**
(45) Date of publication and mention of the grant of the patent: **14.10.2026**
(21) Application number: 24883537.3
(22) Date of filing: 11.07.2024
(51) Int. Cl.: A61F 2/95, A61B 5/00, A61B 5/25, A61B 17/34, A61B 18/14, A61F 2/86, A61N 1/05, A61N 1/36, A61N 1/40, A61N 1/375, A61F 2/90, A61F 2/958, A61F 2/966

(54) **STENT DELIVERY SYSTEM AND DEVICE INCLUDING SAME**
STENTEINFÜHRUNGSSYSTEM UND VORRICHTUNG DAMIT
SYSTÈME DE POSE DE STENT ET DISPOSITIF LE COMPRENANT

(30) Priority: 10.11.2023 KR 20230155685
(43) Date of publication of application: 20.08.2025
(73) Proprietor: Park, Sung Cheol, Seoul 05501 (KR)
(72) Inventor: Park, Sung Cheol, Seoul 05501 (KR)
(74) Representative: Bernsmann, Falk
(86) International application number: PCT/KR2024/009910
(87) International publication number: WO 2025/100685

(56) References cited:
- EP-B1- 3 870 080
- JP-A- 2008 541 882
- JP-A- 2013 066 745
- JP-A- 2018 506 408
- US-A1- 2005 043 779
- US-A1- 2005 137 647
- US-A1- 2011 257 722
- US-A1- 2020 375 766
- US-B1- 6 361 555

## Description

### [Technical field]

The present invention relates to a stent delivery system and a device comprising the same.

### [Background]

The stentrode is a small stent-electrode recording array that is permanently or semi-permanently implanted in the blood vessels of the brain, allowing access via the vasculature without the need for open-brain surgery. This device is currently under clinical trials as a brain-computer interface (BCI) for individuals with paralyzed or missing limbs, or for intracranial brain signal analysis. For example, users can control external devices, including current computer operating systems, using their brain signals or thoughts. Additionally, the device may be used in the future to control other devices, such as powered exoskeletons, robotic substrates, computers, and more.

It was designed by Australian neurologist Thomas Oxley and Australian biomedical engineer Nicholas Opie, who have been developing medical implants using various methods since 2010. Human clinical trials began in August 2019, involving participants with cervical myelopathy (proximal muscle atrophy). Patients who received the implant were able to wirelessly control text writing, email, shopping, and banking through the brain-computer interface using direct thought. This marked the first instance of a brain-computer interface being implanted through the blood vessels, eliminating the need for open-brain surgery.

Such stentrodes are capable of detecting or stimulating electrophysiological signals through the blood vessels and can be used in various applications. However, since their location is limited to inside the blood vessels, the range of signal collection and the quality of the signals are limited.

US 2020/375766 A1 discloses a stent deployed in a brain vessel and a catheter which is inserted through the proximal opening of the stent and steered, by internal structural members of the stent, towards and through the vessel wall. The catheter is thus guided within the lumen of the stent but it not provided with a tip bent toward a side opposite the stent.

### [Detailed description of the invention]

### [Technical problem]

The invention is limited to the appended claims. The present application aims to provide a stent delivery system and a device comprising the same, which can access not only the inside of blood vessels but also the outside of blood vessels through direct electrode wires, enabling more precise detection of physiological signals or activities both inside and outside the blood vessels compared to conventional stentrodes, or applying electrical stimulation.

### [Technical solution]

The present application relates to a stent delivery system comprising a vascular penetration catheter to which a stent combined with an electrode is attached, and the distal end is bent toward the other side.

Preferably, the stent is coupled with a plurality of electrodes, and at least some of the electrodes are configured to directly contact the blood vessel wall upon deployment of the stent.

Preferably, the stent is provided on the balloon of a balloon catheter.

Preferably, the stent delivery system further comprises a hemostatic component located at the tip of the vascular penetration catheter and configured to directly contact the blood vessel wall upon deployment of the stent.

Preferably, the vascular penetration catheter further includes a support facing in the opposite direction to the tip and configured to contact the opposite side of the blood vessel wall when the stent is deployed.

Preferably, the stent delivery system further comprises an electrode wire capable of being positioned outside blood vessel through the vascular penetration catheter.

Preferably, the electrode wire is a directional electrode having an electrode at its tip.

Preferably, the stent delivery system further comprises at least one radiopaque marker attached to the stent or the vascular penetration catheter.

Preferably, the stent delivery system further comprises a stent deployment microcatheter that provides a passage for the vascular penetration catheter to which the stent is attached.

Preferably, the electrode wire has a separable distal end.

Preferably, the stent delivery system further comprises a microarray tube into which the vascular penetration catheter is inserted.

Preferably, the stent delivery system further comprises a microarray tube inserted into the vascular penetration catheter to provide a passage for inserting the electrode wire, penetration wire, or directional cannula.

Preferably, the stent delivery system further comprises a directional cannula inserted into the vascular penetration catheter.

Preferably, the stent delivery system further comprises a directional cannula inserted into the microarray tube.

The present invention relates to a device comprising the stent delivery system.

### [Effect of the invention]

The stent delivery system of the present invention can be used not only to detect or stimulate electrophysiological signals or activities both inside and outside the blood vessels, but also for detecting/recording optical signals and chemical signals, as far as these aspects are encompassed by the appended claims. This allows for more accurate diagnosis and treatment.

The stent delivery system of the present invention can directly detect/record brain electromagnetic signals from outside the blood vessels. Without requiring craniotomy, the system enables the simultaneous acquisition of information from both the inside and outside of blood vessels, as well as brain tissue, making diagnosis and treatment simpler, with less risk, less burden from craniotomy, and minimal pain. The invention facilitates access to the deep brain and areas near the vertebrobasilar artery, which are difficult to reach with conventional craniotomy or trephination, as far as this aspect is encompassed by the appended claims. For example, from the upper side of the middle cerebral artery, access is possible to brain structures such as the basal ganglia, globus pallidus interna, globus pallidus externa, caudate nucleus, subcaudate nucleus, putamen, amygdala, and fornix. From the vertebro-basilar artery, access is possible to the brainstem, subthalamic nucleus, midbrain structures, substantia nigra, and interpeduncular nucleus, as well as the area around posterior cerebral artery. Additionally, brain cortex areas can also be accessed without craniotomy by trans-vascular access.

The invention enables direct access to deep brain structures, which are difficult to approach from the cortex or are located farther and deeper away, allowing for the acquisition of information related to motor, sensory, and memory functions from structures like the deep brain basal ganglia or neural pathways, and it also enables fine electrical stimulation and synapse formation, as far as these aspects are encompassed by the appended claims. Unlike cortical structures, where electrode dislocation may occur due to cerebrospinal fluid leakage or brain shifting caused by gravity, deep brain structures are less prone to movement, minimizing the risk of electrode dislocation and loss of brain electrical signals or neural connections. Thus, compared to conventional cortical approaches, there is less likelihood of the long-term signal loss when accessing deep brain structures.

Furthermore, the invention can prevent damage to the primary motor cortex, which can occur when electrodes are directly inserted into the cortical motor area that controls upstream or primary motor information. In cases where the primary motor cortex is damaged, distal or downstream motor information may also be lost. However, by acquiring motor information from the basal ganglia and similar structures, the invention can prevent damage to the motor cortex.

Additionally, during vascular procedures, the invention allows for the adjustment of the position and direction where the electrode is inserted outside the blood vessel, as far as this aspect is encompassed by the appended claims. The hemostatic stent, which is capable of adjusting its position and direction, can also be used for local hemostasis in the event of vascular injury, excluding the vascular penetration catheter.

### [Brief description of the drawings]

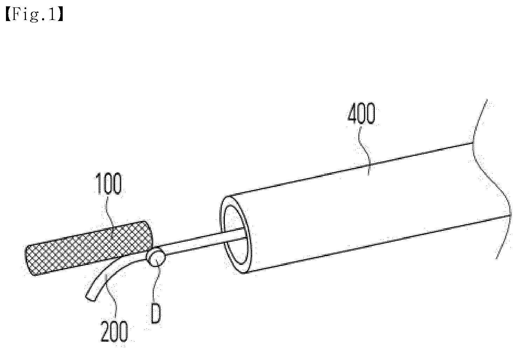

Fig. 1 is a perspective view of a stent delivery system according to one embodiment of the present invention.

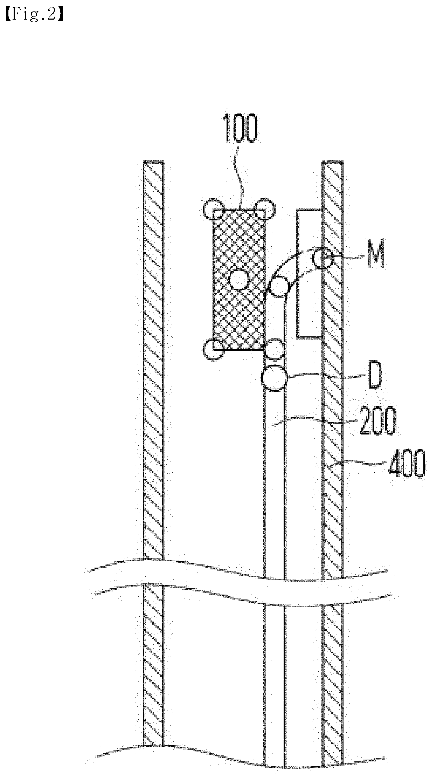

Fig. 2 is a cross-sectional view of a stent delivery system according to one embodiment of the present invention.

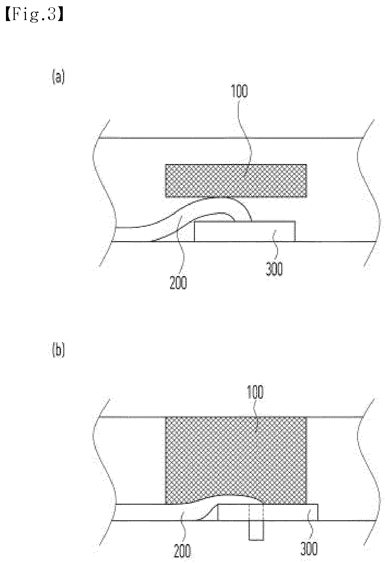

Fig. 3 is a partial cross-sectional view of a stent delivery system according to one embodiment of the present invention, applied inside a blood vessel, showing the state before and after the stent is deployed.

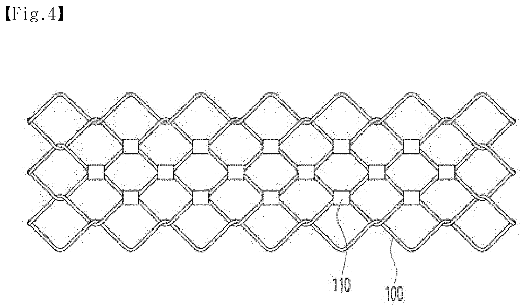

Fig. 4 shows a stent with electrodes attached in the stent delivery system according to one embodiment of the present invention.

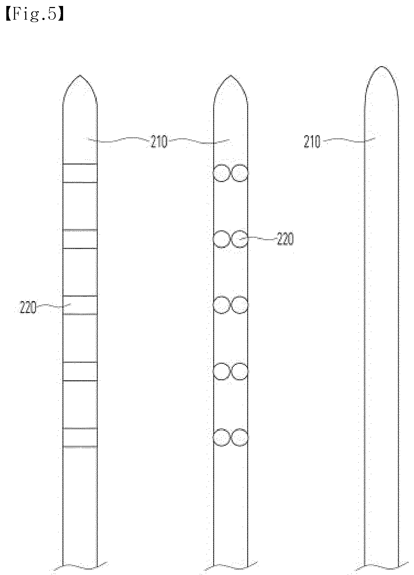

Fig. 5 shows the electrode wire in the stent delivery system according to one embodiment of the present invention.

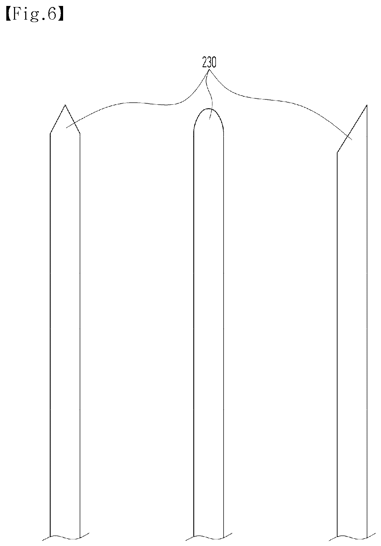

Fig. 6 shows the penetration wire in the stent delivery system according to one embodiment of the present invention.

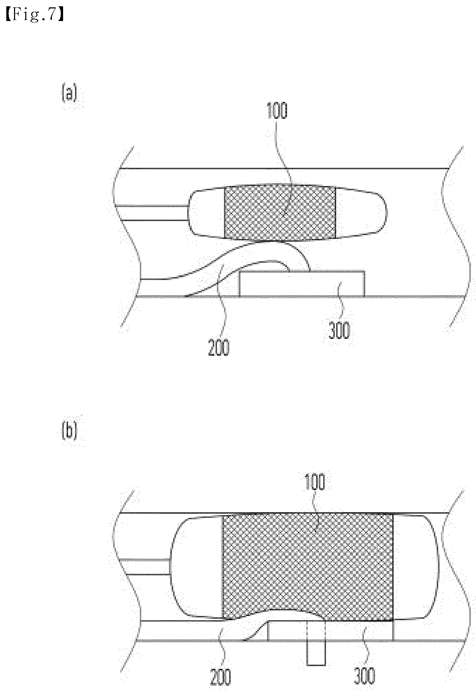

Fig. 7 is an example of stent deployment using a balloon catheter in the stent delivery system according to one embodiment of the present invention.

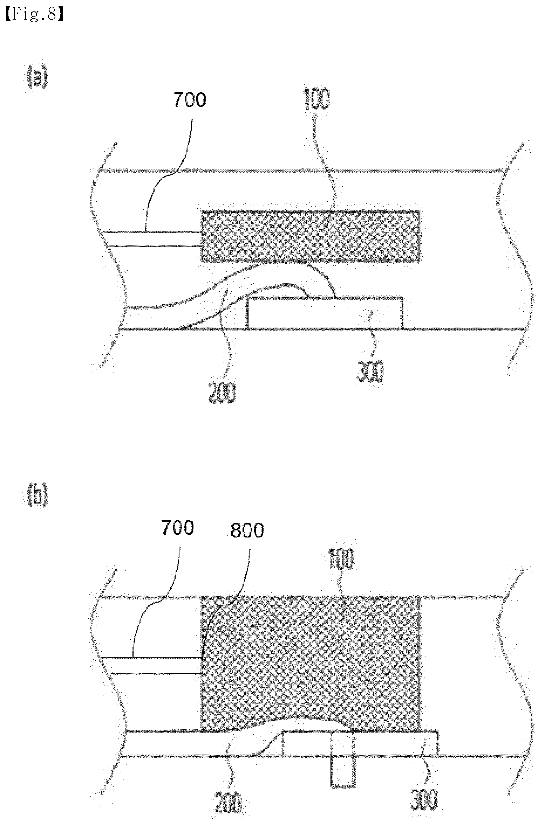

Fig. 8 is an example of stent deployment using a push wire in the stent delivery system according to one embodiment of the present invention.

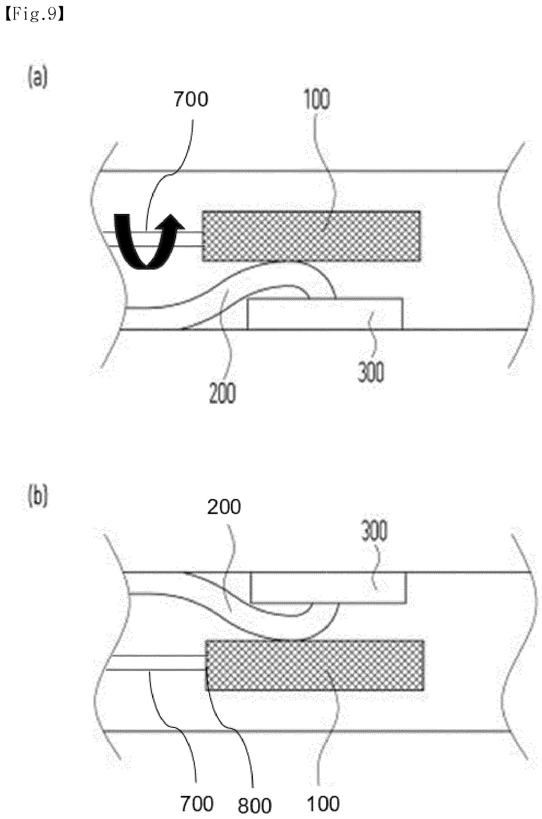

Fig. 9 is an example of rotating the stent using a push wire in the stent delivery system according to one embodiment of the present invention.

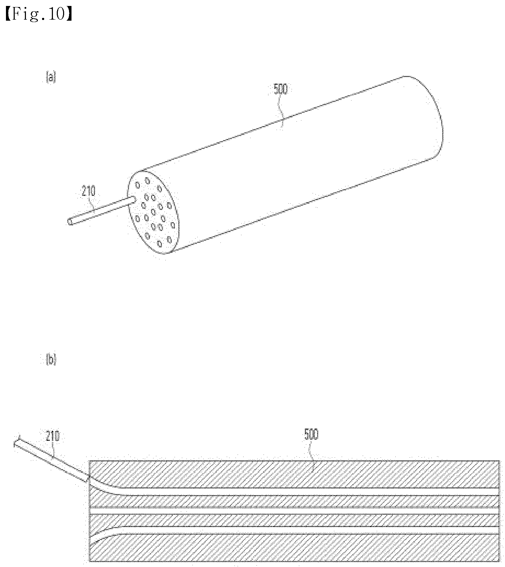

Fig. 10 shows an example of inserting a vascular penetration catheter using a microarray tube in the stent delivery system according to one embodiment of the present invention. The internal passage of the microarray tube may have a curve, which can serve as a directional cannula. The microarray tube can have various forms, such as cylindrical or straight along the longitudinal axis of the stent.

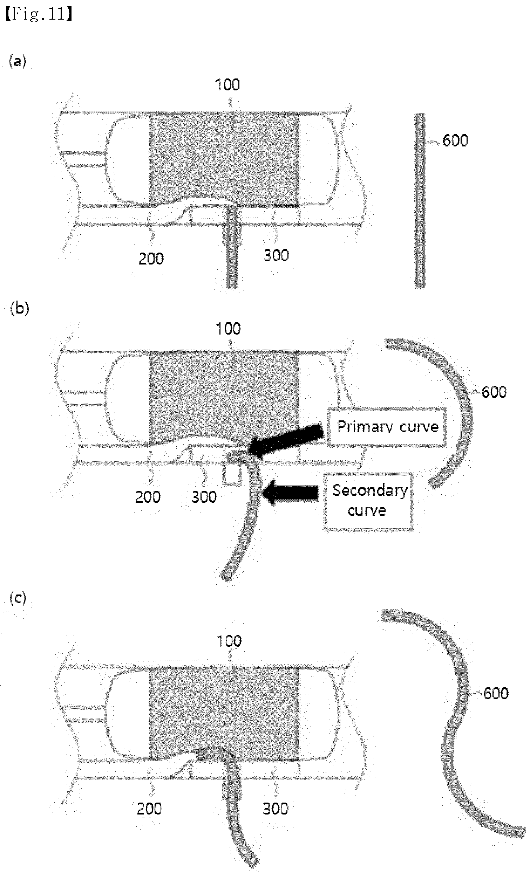

Fig. 11 shows an example of applying a directional cannula in the stent delivery system according to one embodiment of the present invention.

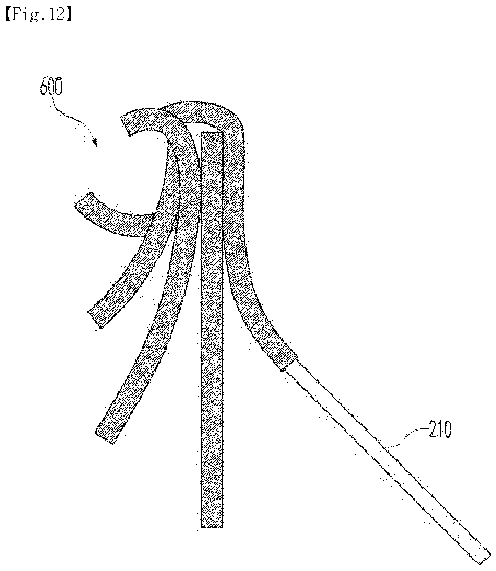

Fig. 12 shows an example of the directional cannula in the stent delivery system according to one embodiment of the present invention. Multiple directional cannulas may be bundled, allowing for the insertion of multiple wires, similar to the microarray tube. Alternatively, the microarray tube may be straight and include multiple vascular penetration catheters and wires.

### [Form for practicing the invention]

Hereinafter, the present invention will be described in detail.

The stent delivery system of the present invention includes a vascular penetration catheter (200) in which a stent (100) having an electrode (110) attached to one side is coupled, and the tip is bent towards the other side. Hereinafter, each component of the stent delivery system of the present invention will be described in detail with reference to the drawings.

The stent (100) may be, for example, in the form of a mesh or a twisted structure. The stent (100) may have an open structure (open cell), a closed structure (closed cell), or a hybrid structure (hybrid, semi-open cell) depending on the purpose and target of use. The material for the stent (100) is not limited and may include any material known in the art, such as stainless steel, cobalt-chromium alloy, nickel-titanium alloy, but is not limited thereto.

The stent (100) has at least one electrode (110) attached. When the stent (100) is deployed, it contacts the blood vessel wall, and at least part of the electrode (110) (at least part of the total electrode (110)) directly contacts the blood vessel wall when the stent (100) is deployed, allowing it to record electrical signals from surrounding tissues, or perform functions such as stimulation or destruction.

The stent (100) may have any appropriate number of electrodes (110), and their arrangement and spacing are not limited. The electrode (110) may be attached to the stent (100) either directly or indirectly by any known method in the field. The material of the electrode (110) may be any electrically conductive material known in the art without limitation, including but not limited to platinum, platinum-iridium, nickel-cobalt alloy, or gold.

A radiopaque marker may be attached to the stent (100), allowing the deployment location and position of the stent (100) to be identified. The radiopaque marker may be used in an appropriate number and position depending on its purpose. The type of radiopaque marker is not limited, and any known in the art can be used without limitation.

The stent (100) can be deployed by various known methods. For example, it may be fixed with a stent deployment wire (700), and deployed by retracting a microcatheter (400) for stent deployment (push and pull methods), or it may be mounted on the balloon of a balloon catheter and deployed by inflating the balloon (balloon-expandable stent method).

The stent deployment wire (700) may be connected to the stent (100). In addition, the stent (100) may be connected to the vascular penetration catheter (200) and the hemostatic component (300), so that by adjusting the position of the stent deployment wire (700) or rotating it, the position of the stent (100), vascular penetration catheter (200), and hemostatic component (300) can also be adjusted or rotated. This allows the selection of the penetration site along the circumference of the blood vessel, targeting a desired anatomical location or structure outside the blood vessel.

Rotation and position adjustment may be performed before or after stent (100) deployment, but it may be difficult or risky after deployment due to resistance from the blood vessel wall, so it can be done inside the microcatheter (400) before stent deployment.

The stent deployment wire (700) can be detached from the stent (100) at the connection point (800) by electrical stimulation or physical force, and then retracted proximally and removed from the vessel. In cases where rotation is difficult due to vascular structure, highly curved blood vessels, microcatheter structure, or friction between the stent and the microcatheter, structures for vascular penetration such as the vascular penetration catheter (200) and the hemostatic component (300) may be pre-arranged in multiple directions around the circumference of the stent (100) and microcatheter (400), allowing selection of the penetration site or implementation of vascular penetration electrode insertion in multiple directions.

The stent (100) is attached to one side of the vascular penetration catheter (200) described below. The vascular penetration catheter (200) provides a pathway for the electrode wire (210), optical fibers, and other components described below.

The vascular penetration catheter (200) is bent so that its tip faces the other side, the direction perpendicular, near perpendicular or slanted to the blood vessel wall, allowing it to position against the blood vessel wall after the stent (100) is deployed. The angle may be perpendicular to the other side and can be adjusted as needed depending on the purpose, location, or blood vessel. It is preferable for the vascular penetration catheter (200) to be cylindrical, but it is not limited thereto.

If necessary, the vascular penetration microcatheter may further include a support that faces the one side, and can be configured to contact the opposite blood vessel wall when the stent (100) is deployed. A radiopaque marker may be attached to the vascular penetration catheter (200), allowing identification of the location and angle of vascular penetration as the electrode wire (210) moves through the pathway. The radiopaque marker may be used in an appropriate number and position depending on its purpose. The type of radiopaque marker is not limited and any known in the art can be used without limitation.

The radiopaque marker may have various shapes, such as an ellipse, circle, cross, two connected triangles, or a combination of a triangle or cross with a circle or curve. The radiopaque marker can be attached to any component for marking, such as the stent (100), vascular penetration catheter (200), electrode wire (210), hemostatic component (300), or stent deployment microcatheter (400). Since the stent (100) and other components can be rotated or adjusted in position, the radiopaque marker can also be adjusted or rotated accordingly.

The radiopaque marker may be permanently located on the stent (100), vascular penetration catheter (200), electrode wire (210), or hemostatic component (300) within the blood vessel wall, or it may be removed with the stent deployment microcatheter (400) after stent deployment.

The electrode wire (210) may be preloaded within the vascular penetration catheter (200) or may be inserted later through the internal pathway after positioning the vascular penetration catheter (200) at the desired site within the blood vessel. As mentioned earlier, the tip of the vascular penetration catheter (200) is bent so that it can be directed against the blood vessel wall when deployed with stent (100). At this time, the electrode wire (210) can move through the internal pathway of the vascular penetration catheter (200) and penetrate the blood vessel wall, allowing electrical signals outside the blood vessel to be detected or electrical stimulation to be applied outside the blood vessel.

In addition to the electrode wire (210), the vascular penetration catheter (200) can accommodate various components. For example, a penetration wire (230) for penetrating the blood vessel wall, optical fibers for detecting or recording light signals, conduits for delivering drugs, organic or inorganic materials, or devices or organic-inorganic composites for nerve junctions can be applied.

The electrode wire (210) may penetrate the blood vessel wall directly, or the penetration wire (230) may penetrate the blood vessel wall first, followed by the application of the electrode wire (110). The tip of the penetration wire (230), which contacts the blood vessel wall, would have sufficient stiffness and sharpness for vascular penetration. The tip may have various shapes as long as it is capable of penetrating the blood vessel.

The electrode wire (210) itself may be in the form of a wire electrode (110), or it may be a directional electrode (110) with at least one electrode (110) on its tip. Any electrically conductive material may be used for the electrode wire (210) or its electrode (110) without limitation, including but not limited to platinum, platinum-iridium, nickel-cobalt alloy, or gold.

If the stent deployment wire (700) is directly connected to the electrode wire (210), vascular penetration catheter (200), or stent (100), these can later be separated by electrical or mechanical force at the distal end and removed proximally. The electrode (110) can also be separated from the distal end by electrical stimulation if necessary.

When the electrode wire (210) penetrates the blood vessel wall, bleeding may occur, so the stent delivery system may further include a hemostatic component (300) located at the tip of the vascular penetration catheter (200). The hemostatic component (300) may cover the remaining part of the vascular penetration area after the electrode wire (210) penetrates the blood vessel wall.

The hemostatic component (300) may include a hemostatic pad or any other hemostatic material used in the field without limitation. It may also include a hemostatic drug applied to the hemostatic component (300). For example, a coagulation factor, vascular strengthening agent, vasoconstrictor, or physical coagulant may be used, but it is not limited thereto.

The hemostatic component (300) may be attached or connected to the tip of the vascular penetration catheter (200) by any known method or means in the field. It may have a wide shape to cover large areas of vascular damage (e.g., vascular damage, vascular wall loss, ruptured aneurysm, blister aneurysm, vascular dissection), or it may be used to locally stop bleeding. Like the vascular penetration catheter (200), the hemostatic component (300) can also be rotated and adjusted in position by the connection between the stent (100) and the stent deployment wire (700), and multiple hemostatic components may be pre-arranged for hemostasis.

The stent delivery system of the present invention may further include a stent deployment microcatheter (400) that provides a pathway for the vascular penetration catheter (200) to which the stent (100) is attached. The stent deployment microcatheter (400) provides a pathway for the vascular penetration catheter (200) to which the stent (100) is attached, and may consist of a single stage or, if necessary, may include an intermediate conduit (410), guiding conduit (420), or the like to form two or three stages. This may follow configurations and forms known in the field.

If the stent deployment microcatheter (400) consists of multiple stages, the outer stage will have a larger diameter, and the inner stage can be moved forward or backward.

The aforementioned radiopaque marker may be attached to any component of the stent delivery system, including the catheter, wire, and others. One or more radiopaque markers can be attached to each component, and all radiopaque markers may be the same or different.

The stent delivery system of the present invention may further include a microarray tube (500). The microarray tube (500) may be located inside or outside the vascular penetration catheter (200). If the microarray tube (500) is located inside the vascular penetration catheter (200), it can provide a pathway for one or more electrode wires (210), penetration wires (230), cannulas (600) for directional adjustment, and the like.

The electrode wire (210) or a device inserted in the same manner may have a passage for delivering drugs, organic materials, or inorganic materials inside, and this passage may have one or multiple porous sections. When the microarray tube (500) is positioned outside the vascular penetration catheter (200), it can provide a passage for one or multiple vascular penetration catheters (200). The microarray tube (500) may be a hollow tube with one or more holes. These holes can provide pathways for the insertion of wires, catheters, or cannulas for directional adjustment. The number of holes may be one, more than 10, more than 100, etc., and is not limited in number.

In a different manner from the microarray tube (500), multiple vascular penetration catheters (200) and hemostatic component (300) complexes may be arranged along the circumference of the stent (100), and these can be selected and used in various directions. The stent delivery system of the present invention may further include a directional adjustment cannula (600). The directional adjustment cannula (600) allows for fine adjustment of the direction of the electrode wire (210), penetration wire (230), etc. The directional adjustment cannula (600) may be inserted inside the vascular penetration catheter (200) or inside the microarray tube (500).

The directional adjustment cannula (600) may have a flexible part for direction adjustment and a rigid part. The directional adjustment cannula (600) may be made of metal such as nitinol, stainless steel, or plastic, or a combination of these materials. The directional adjustment cannula (600) can move along the curved path of the vascular penetration catheter (200), which is bent in the same direction as the tip of the catheter (200) aimed at the blood vessel wall. The direction of the directional adjustment cannula (600) can be set based on the two-dimensional or three-dimensional curvature relationship between the primary curve slightly proximal to the tip and the secondary curve of the tip of the cannula (600) as it exits the vascular penetration catheter (200). This directional setting can be achieved using two or more combinations of these curves. For example, if the tip of the directional adjustment cannula (600) is straight, the cannula (600) will proceed in the same direction as the vascular penetration catheter (200).

If the secondary curve at the tip of the directional adjustment cannula (600) is bent in the same direction as the primary curve proximal to it, the cannula (600) will be positioned in a more curved direction along the primary curve. If the secondary curve at the tip of the directional adjustment cannula (600) is bent in the opposite direction of the primary curve, the cannula (600) will be positioned in a more curved direction opposite the primary curve. By using this fine curvature of the directional adjustment cannula (600), the direction and position of the electrode wire (210) being inserted can be adjusted.

These curved shapes can be combined into three-dimensional relationships, such as up-and-down, left-and-right, pig-tail shapes, etc., for various directional settings beyond the two-dimensional relationship between the primary and secondary curves. The directional adjustment cannula (600) may be inserted into the vascular penetration catheter (200) to set the external or internal direction of the electrode wire (210), etc. Various curved types of directional adjustment cannulas (600) may be prepared in advance, or the shape may be adjusted during the procedure through mechanical or thermal stimulation. The curve can also be adjusted using external magnetic fields or electrical stimulation inside the directional adjustment cannula (600).

Multiple directional adjustment cannulas (600) can be used together inside the microarray tube (500). The directional adjustment cannula (600) may be removed after the insertion of microelectrodes into brain tissue, or it may be left in place for directional maintenance, or separated and removed at the proximal end through electrical or mechanical stimulation. In traditional stents, only the longitudinal position along the vascular direction of the stent was considered, but the position in the 360-degree direction around the blood vessel wall was not considered. In the present invention, since the vascular penetration catheter (200) must be positioned in a direction close to the desired brain tissue structure, the position must be determined based on the circumferential 360-degree rotational direction of the blood vessel wall.

The tip of the vascular penetration catheter (200), which is bent in the same direction as the distal end of the stent, may have a radiopaque marker attached to the wire end or the wire itself may be made radiopaque. These primary curve-shaped or question-mark-shaped markers are positioned outside the distal end of the stent deployment microcatheter (400), indicating the direction of the vascular penetration catheter (200) tip, and when the stent is deployed, the marker adheres to the blood vessel wall and marks the stent location while deploying without obstructing blood flow.

In another method, if the diameter of the stent deployment microcatheter (400) is large or if the resolution of the cerebrovascular imaging equipment is sufficiently high, the direction can be confirmed by directly marking the distal tip of the vascular penetration catheter (200), the stent marker, or the stent deployment microcatheter (400). Alternatively, it may be connected to the stent deployment wire (700) and protrude outside the distal end of the stent deployment microcatheter (400). In another method, a side hole may be created in the stent deployment microcatheter (400) near the position of the vascular penetration catheter (200), and a flexible wire with a radiopaque marker may be protruded through the hole to mark the direction. The marker can be used to estimate the direction of the vascular penetration catheter (200), determining which direction of the 360-degree circumferential blood vessel wall the penetration will occur after deployment.

These rotational direction markers protruding from the side hole may be connected to the stent, vascular penetration catheter (200), or stent deployment microcatheter (400), and after deployment, they can naturally be pressed by the stent and adhere to the blood vessel wall. Additionally, these radiopaque markers may be positioned slightly away from the hemostatic component around the vascular penetration catheter (200) to prevent interference with the hemostasis process by the hemostatic component. The complex of the stent, vascular penetration catheter (200), and hemostatic component (300) can be directly connected to a proximal push/pull/rotation wire, allowing the vascular penetration catheter (200) to rotate before stent deployment, adjusting the radial penetration direction within the 360-degree circumferential direction perpendicular to the vascular progression direction.

Through push/pull/rotation parallel to the vascular direction, components such as microwires or supermicrocatheters inside the microcatheter of the vascular penetration catheter (200), or microelectrode array chips can penetrate outside the blood vessel and adjust the anatomical structure encountered when exiting the longitudinal (vascular length direction) and circumferential (vascular circumference) directions. The longitudinal push/pull and circumferential rotation wires controlled from the proximal end must be physically connected to the stent for directional adjustment before deployment, but after deployment, they may be separated and removed through electrical, mechanical, or pressure methods.

By adding directional adjustment through curves in the vascular penetration catheter (200), electrode wire (210), and penetration wire (230), it is possible to target micro-targets in cerebral blood vessels or outside the vessels. Alternatively, multiple vascular penetration catheters (200), hemostatic components (300), etc., may be arranged in various directions in advance to select the penetration direction.

Using the stent positioning and direction change technology along with the stent with a hemostatic component (300), the stent delivery system can be used for local hemostasis of vascular damage while excluding parts such as the vascular penetration catheter (200) and electrode. In conventional grafted stents, most of the blood vessel was occluded, increasing the risk of infarction or cerebral infarction due to side vessel occlusion. However, the system of the present invention can selectively and safely stop bleeding by partially occluding only the localized area of vascular damage.

The size and position of the hemostatic component (300) can be adjusted according to the purpose of use and the extent of vascular damage. For example, in the case of a blister aneurysm, it can be made to cover more than half of the blood vessel circumference (about 180 degrees) and 1 cm or more in the longitudinal direction to include a blister aneurysm area. For longer vascular dissection bleeding or dissecting aneurysms, it can be made in a longer shape. The hemostatic component (300) may include thrombin or other substances to promote hemostasis, along with sponge-like materials that are compressed around the damaged area by the stent for structural support and vascular occlusion.

The wide-range hemostatic component (300) for vascular hemostasis may be customized for the patient or lesion using methods such as 3D printing, or it may be made in various pre-formed shapes. The system of the present invention can be used for internal vascular electrode arrays and external vascular electrode insertion in arteries and veins. Hemostasis is achieved by applying pressure with the stent and the hemostatic component from inside to outside, and since arterial walls are thicker than venous walls, they can provide better support for hemostasis. Also, since arterial blood flow is faster and less stagnant than venous blood flow, the risk of thrombus formation may be lower.

Additionally, the present invention relates to a device including the aforementioned stent delivery system. The device of the present invention can stimulate or detect the activity of tissue adjacent to the blood vessel at the target site, and can enable sensing, recording, or destruction by electrical stimulation (radiofrequency stimulation). It can also deliver drugs, measure external chemical compositions, or form junctions with external cell receptors.

The device of the present invention may further include additional components to enable electrical stimulation, detection, etc., using the stent delivery system. For example, it may include a current and/or voltage source, a battery and/or capacitor or charge/energy storage component, a circuit, an amplifier, a control unit, etc., but is not limited thereto. The control unit, for example, may receive information from the electrode (110) and electrode wire (210) of the stent delivery system or generate and transmit control signals for the catheter.

### [Explanation of Symbols]

100: Stent
110: Electrode
200: Vascular Penetration Catheter
210: Electrode Wire
220: Electrode (Electrode Wire' s Electrode)
230: Penetration Wire
300: Hemostatic Component
400: Stent Deployment Microcatheter
410: Intermediate Conduit
420: Guiding Conduit
500: Microarray Tube
600: Directional Adjustment Cannula
700: Stent Deployment Wire
800: Connection Point
D: Detachable point marker, an example location
M: Radiopaque Marker

## Claims

1. A stent delivery system comprising a vascular penetration catheter (200) and a stent (100), wherein the stent (100), having an electrode (110) attached to one side of the stent (100), is attached to one side of the vascular penetration catheter (200), and the tip of the vascular penetration catheter (200) is bent toward the side of the vascular penetration catheter (200) opposite to the stent (100),
**characterized in that**
the stent (100) is configured to push the vascular penetration catheter (200) against a blood vessel wall during deployment of the stent (100) in a blood vessel, such that the vascular penetration catheter (200) can guide a penetration wire (230) or an electrode wire (210) through an internal pathway of the vascular penetration catheter (200) to penetrate the blood vessel wall.

2. The stent delivery system according to claim 1, wherein the stent (100) is coupled with a plurality of electrodes (110), and at least some of the electrodes (110) are configured to directly contact the blood vessel wall upon deployment of the stent (100).

3. The stent delivery system according to claim 1, wherein the stent (100) is provided on the balloon of a balloon catheter.

4. The stent delivery system according to claim 1, further comprising a hemostatic component (300) located at the tip of the vascular penetration catheter (200) and configured to directly contact the blood vessel wall upon deployment of the stent (100).

5. The stent delivery system according to claim 1, further comprising an electrode wire (210) capable of being positioned outside the blood vessel through the vascular penetration catheter (200).

6. The stent delivery system according to claim 5, wherein the electrode wire (210) is a directional electrode having an electrode (220) at its tip.

7. The stent delivery system according to claim 1, further comprising at least one radiopaque marker (M) attached to the stent (100) or the vascular penetration catheter (200).

8. The stent delivery system according to claim 1, further comprising a stent deployment microcatheter (400) that provides a passage for the vascular penetration catheter (200) to which the stent (100) is attached.

9. The stent delivery system according to claim 5, wherein the electrode wire (210) has a separable distal end.

10. The stent delivery system according to claim 1, further comprising a microarray tube (500) into which the vascular penetration catheter (200) is inserted.

11. The stent delivery system according to claim 1, further comprising a microarray tube (500) that provides a passage for the insertion of an electrode wire (210), a penetration wire (230), or a directional adjustment cannula (600) inside the vascular penetration catheter (200).

12. The stent delivery system according to claim 1, further comprising a directional adjustment cannula (600) inserted into the vascular penetration catheter (200).

13. The stent delivery system according to claim 11, further comprising a directional adjustment cannula (600) inserted into the microarray tube (500).

14. A device comprising the stent delivery system according to any one of claims 1 to 13.

## Patentansprüche

1. Stent-Einführsystem, bestehend aus einem Gefäßpenetrationskatheter (200) und einem Stent (100), wobei der Stent (100), an dessen einer Seite eine Elektrode (110) angebracht ist, an einer Seite des Gefäßpenetrationskatheters (200) befestigt ist, und die Spitze des Gefäßpenetrationskatheters (200) in Richtung der dem Stent (100) abgewandten Seite des Gefäßpenetrationskatheters (200) gebogen ist ,
**dadurch gekennzeichnet, dass**
der Stent (100) so ausgebildet ist, dass er den Gefäßpenetrationskatheter (200) während des Entfaltens des Stents (100) in einem Blutgefäß gegen eine Blutgefäßwand drückt, sodass der Gefäßpenetrationskatheter (200) einen Penetrationsdraht (230) oder einen Elektrodendraht (210) durch einen inneren Pfad des Gefäßpenetrationskatheters (200) führen kann, um die Blutgefäßwand zu durchdringen.

2. Stent-Einführsystem nach Anspruch 1, wobei der Stent (100) mit einer Mehrzahl von Elektroden (110) verbunden ist, und zumindest einige der Elektroden (110) so konfiguriert sind, dass sie bei der Entfaltung des Stents (100) direkten Kontakt mit der Blutgefäßwand herstellen.

3. Stent-Einführsystem nach Anspruch 1, wobei der Stent (100) auf dem Ballon eines Ballonkatheters bereitgestellt ist.

4. Stent-Einführsystem nach Anspruch 1, das ferner eine hämostatische Komponente (300) umfasst, die an der Spitze des Gefäßpenetrationskatheters (200) angeordnet und so ausgebildet ist, dass sie bei der Entfaltung des Stents (100) direkt mit der Blutgefäßwand in Kontakt kommt.

5. Stent-Einführsystem nach Anspruch 1, das ferner einen Elektrodendraht (210) umfasst, der durch den Gefäßpenetrationskatheter (200) außerhalb des Blutgefäßes positioniert werden kann.

6. Stent-Einführsystem nach Anspruch 5, wobei der Elektrodendraht (210) eine Richtungselektrode ist, die an ihrer Spitze eine Elektrode (220) aufweist.

7. Stent-Einführsystem nach Anspruch 1, das ferner mindestens eine röntgendichte Markierung (M) umfasst, die an dem Stent (100) oder an dem Gefäßpenetrationskatheter (200) angebracht ist.

8. Stent-Einführsystem nach Anspruch 1, das ferner einen Stent-Entfaltungs-Mikrokatheter (400) umfasst, der einen Durchgang für den Gefäßpenetrationskatheter (200) bereitstellt, an dem der Stent (100) befestigt ist.

9. Stent-Einführsystem nach Anspruch 5, wobei der Elektrodendraht (210) ein abtrennbares distales Ende aufweist.

10. Stent-Einführsystem nach Anspruch 1, das ferner ein Mikroarray-Rohr (500) umfasst, in das der Gefäßpenetrationskatheter (200) eingeführt ist.

11. Stent-Einführsystem nach Anspruch 1, das ferner ein Microarray-Rohr (500) umfasst, das einen Durchgang für das Einführen eines Elektrodendrahts (210), eines Penetrationsdrahts (230) oder einer Richtungsjustierkanüle (600) in dem Gefäßpenetrationskatheter (200) bereitstellt.

12. Stent-Einführsystem nach Anspruch 1, das ferner eine in den Gefäßpenetrationskatheter (200) eingeführte Richtungsjustierkanüle (600) umfasst.

13. Stent-Einführsystem nach Anspruch 11, das ferner eine in das Mikroarray-Rohr (500) eingeführte Richtungsjustierkanüle (600) umfasst.

14. Vorrichtung, die das Stent-Einführsystem gemäß einem der Ansprüche 1 bis 13 umfasst.

## Revendications

1. Système de pose de stent comprenant un cathéter de pénétration vasculaire (200) et un stent (100), dans lequel le stent (100), comportant une électrode (110) fixée sur l'un de ses côtés, est fixé sur l'un des côtés du cathéter de pénétration vasculaire (200), et la pointe du cathéter de pénétration vasculaire (200) est courbé vers le côté du cathéter de pénétration vasculaire (200) opposé au stent (100),
**caractérisé en ce que**
le stent (100) est configuré pour pousser le cathéter de pénétration vasculaire (200) contre une paroi vasculaire lors du déploiement du stent (100) dans un vaisseau sanguin, de telle sorte que le cathéter de pénétration vasculaire (200) puisse guider un fil de pénétration (230) ou un fil d'électrode (210) à travers une voie interne du cathéter de pénétration vasculaire (200) afin de pénétrer la paroi du vaisseau sanguin.

2. Système de pose de stent selon la revendication 1, dans lequel le stent (100) est couplé à une pluralité d'électrodes (110), et au moins certaines des électrodes (110) sont configurées pour entrer en contact direct avec la paroi du vaisseau sanguin lors du déploiement du stent (100).

3. Système de pose de stent selon la revendication 1, dans lequel le stent (100) est monté sur le ballonnet d'un cathéter à ballonnet.

4. Système de pose de stent selon la revendication 1, comprenant en outre un composant hémostatique (300) situé à la pointe du cathéter de pénétration vasculaire (200) et configuré pour entrer en contact direct avec la paroi du vaisseau sanguin lors du déploiement du stent (100).

5. Système de pose de stent selon la revendication 1, comprenant en outre un fil d'électrode (210) pouvant être positionné à l'extérieur du vaisseau sanguin à travers le cathéter de pénétration vasculaire (200).

6. Système de pose de stent selon la revendication 5, dans lequel le fil d'électrode (210) est une électrode directionnelle comportant une électrode (220) à son extrémité.

7. Système de pose de stent selon la revendication 1, comprenant en outre au moins un marquage radio-opaque (M) fixé au stent (100) ou au cathéter de pénétration vasculaire (200).

8. Système de pose de stent selon la revendication 1, comprenant en outre un microcathéter de déploiement de stent (400) qui fournit un passage pour le cathéter de pénétration vasculaire (200) auquel le stent (100) est fixé.

9. Système de pose de stent selon la revendication 5, dans lequel le fil d'électrode (210) comporte une extrémité distale séparable.

10. Système de pose de stent selon la revendication 1, comprenant en outre un tube à micro-arrray (500) dans lequel est inséré le cathéter de pénétration vasculaire (200).

11. Système de pose de stent selon la revendication 1, comprenant en outre un tube à micro-array (500) qui fournit un passage pour l'insertion d'un fil d'électrode (210), d'un fil de pénétration (230) ou d'une canule d'ajustement directionnel (600) à l'intérieur du cathéter de pénétration vasculaire (200).

12. Système de pose de stent selon la revendication 1, comprenant en outre une canule d'ajustement directionnel (600) insérée dans le cathéter de pénétration vasculaire (200).

13. Système de pose de stent selon la revendication 11, comprenant en outre une canule d'ajustement directionnel (600) insérée dans le tube à micro-array (500).

14. Dispositif comprenant le système de pose de stent selon l'une quelconque des revendications 1 à 13.
